# EUROPEAN PATENT APPLICATION

(11) **EP 4 509 610 A1**
(43) Date of publication of application: **19.02.2025**
(21) Application number: 23787661.0
(22) Date of filing: 11.04.2023
(51) Int. Cl.: C12P 13/04, C12N 5/10, C12N 9/04, C12N 9/88, C12N 15/52, C12N 15/63

(54) **METHOD FOR PREPARING KETO ACIDS, AND USE OF SAME IN PREPARATION OF AMINO ACIDS OR AMINO ACID DERIVATIVES**

(30) Priority: 11.04.2022 CN 202210371436
(71) Applicant: Mint Biotechnologies Co., Ltd., Hangzhou, Zhejiang 310012 (CN)
(72) Inventor: NIE, Mengzhen, Hangzhou, Zhejiang 310012 (CN); ZHANG, Kechun, Hangzhou, Zhejiang 310012 (CN)
(74) Representative: Gille Hrabal Partnerschaftsgesellschaft mbB Patentanwälte
(86) International application number: PCT/CN2023/087476
(87) International publication number: WO 2023/198017

(57) **Abstract**

The present invention relates to the technical field of genetic engineering, in particular to a method for preparing a keto acid, in which an enzymatic reaction is carried out by using glycine and an alcoholic organic substance as substrates; during the enzymatic reaction process, the alcoholic organic substance is converted into an aldehyde organic substance, glycine and the aldehyde organic substance are converted into a β-hydroxy-α-amino acid, and then the β-hydroxy-α-amino acid is converted into a keto acid. Also disclosed in the present invention is the use of the preparation method for a keto acid in the preparation of amino acids. The number of enzymes used in the present invention is much less than that of enzymes used in a natural synthesis route, so that the production cost is low; an artificial metabolism platform for keto acids is established and can produce multiple important keto acids, such as phenylpyruvic acid, 4-methyl-2-oxopentanoic acid, pyruvic acid and 2-oxo-butyric acid; the method features simple operation, high yield, high substrate utilization rate and low pollution.

## Description

### CROSS-REFERENCE TO RELATED APPLICATIONS

The present disclosure claims priority to Chinese Patent Application No. 202210371436.6 filed to China National Intellectual Property Administration on Apr. 11, 2022 and entitled "METHOD FOR PREPARING KETO ACIDS, AND USE OF SAME IN PREPARATION OF AMINO ACIDS OR AMINO ACID DERIVATIVES", which is incorporated herein by reference in its entirety.

### TECHNICAL FIELD

The present disclosure relates to the technical field of bioengineering, and particularly relates to a method for preparing a keto acid and use of the method for manufacturing an amino acid.

### BACKGROUND

Amino acids are the basic unit of biological functional macromolecular proteins, and play an important role in life activities of humans and animals as well as in nutrition and health. An amino acid is an organic compound comprising a basic amino group (-NH₂) and an acidic carboxyl group (-COOH), and the amino group and the carboxyl group are attached to the same carbon atom, with a chemical general formula of RCHNH₂COOH. According to the degree of need for the human body, amino acids can be divided into essential amino acids and non-essential amino acids. In addition to biosynthesized natural amino acids, amino acids also include artificially synthesized unnatural amino acids. Amino acid products have a wide range of applications in medical/health care, food (flavorings), feed additives, cosmetics, chemical synthesis, etc., and have become a major global industry.

Currently, the main production methods for amino acids in the world include fermentation method, chemical synthesis method, hydrolysis method, and chemical synthesis-enzymatic method. The fermentation method is a production method that utilizes the inherent ability of microorganisms to synthesize various amino acids, and selects and breeds various defective and resistant mutant strains through strain mutagenesis and other treatments, in order to relieve the feedback and repression in the metabolism pathway, and achieve the overproduction of a certain amino acid. The inevitable disadvantages of the fermentation method for producing amino acids are the difficulty of controlling the production and accumulation of various non-target amino acids, long production period, high difficulty of purification, and high cost. The chemical synthesis method is a method for producing amino acids by means of complicated reactions between various organic substances. Although various natural and unnatural amino acids can be produced by the chemical synthesis method, most of the resulting amino acids are optical racemates (comprising two kinds of optical isomers, D and L), which greatly restricts the industrial value. Meanwhile, the chemical synthesis method also has the problems of complicated reactions, numerous steps, and environmental pollution. The hydrolysis method is a process to obtain various amino acids by using protein-rich materials such as hair as starting materials, subjecting the same to hydrolysis with acid, alkali, or proteolytic enzyme, and then performing separation and purification. The hydrolysis method, with richer starting materials, is relatively easy to put into production, but has low yield, high cost, and relatively serious pollution to the environment.

Most of the amino acids can be synthesized using keto acids via a one-step reaction with transaminases, so the production and preparation of keto acids are of great importance. Currently, the problems in the preparation of keto acids are use of toxic catalysts and the phenomenon of low yield and transformation rate. US Patent No. US08153839B2 discloses a method for synthesizing a keto acid or an amino acid by hydration of an acetylene compound, which takes advantage of the hydration of a metal salt and a transition metal coordination complex to allow the synthesis of a keto acid (including a keto acid and a keto acid derivative) from acetylene-carboxylic acid under mild conditions without using an extremely harmful mercury catalyst, and which can easily synthesize an amino acid (including an amino acid and an amino acid derivative) from the synthesized keto acid (including a keto acid and a keto acid derivative) by subsequent reductive amination in the same container. However, the operation thereof is complicated, the production process is difficult to control, and the yield and transformation rate are low, making it difficult to achieve industrial mass production.

Therefore, the present disclosure aims to seek a breakthrough in technological innovation for the preparation of keto acids, improve the yield of a plurality of natural amino acids or unnatural amino acids and derivatives in all aspects, and enhance the competitiveness of the product in the market.

### SUMMARY

The first technical problem to be solved by the present disclosure is to provide a method for preparing a keto acid with high substrate utilization rate, low pollution, high yield, and low production cost.

The second technical problem to be solved by the present disclosure is to provide a method for preparing a glycol organic substance with high substrate utilization rate, low pollution, high yield, and low production cost.

The third technical problem to be solved by the present disclosure is to be able to produce a plurality of important keto acids: phenylpyruvic acid, 4-methyl-2-oxopentanoic acid, pyruvic acid, 2-oxobutyric acid, etc., and the pathway can be applied to the production of other keto acids and amino acids.

The technical problem solved by the present disclosure is realized by adopting the following technical solutions:
In a first aspect, provided is a method for preparing a keto acid, wherein an enzymatic reaction is performed using glycine and an alcoholic organic substance as substrates, wherein during the enzymatic reaction process, the alcoholic organic substance is converted into an aldehyde organic substance, the glycine and the aldehyde organic substance are converted into a β-hydroxy-α-amino acid, and then the β-hydroxy-α-amino acid is converted into the keto acid.

Preferably, the keto acid prepared comprises, but is not limited to, a keto acid having the following general formula: 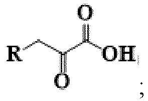 wherein the structural formula of R may be 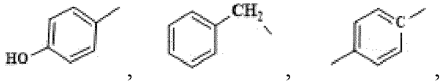 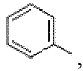 (CH₃)₂CH₂-, (CH₃)C-, CH₃-, 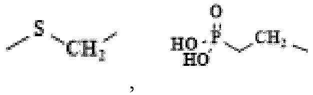 , etc.

Further, an enzymatic reaction is performed using glycine and an alcoholic organic substance as substrates and using an enzyme produced by overexpression of a first recombinant microorganism comprising L-aldolase and first dehydratase genes and a second recombinant microorganism comprising a dehydrogenase as a catalyst, wherein the alcoholic organic substance is converted into an aldehyde organic substance in the presence of the dehydrogenase, the glycine and the aldehyde organic substance are converted into a β-hydroxy-α-amino acid under the independent catalysis of the L-aldolase, and the β-hydroxy-α-amino acid generates the keto acid under the catalysis of the first dehydratase;
or an enzymatic reaction is performed using an enzyme produced by overexpression of a third recombinant microorganism comprising a D-aldolase gene, a racemase gene, and a second dehydratase gene and a second recombinant microorganism comprising the dehydrogenase as a catalyst, wherein the alcoholic organic substance is converted into an aldehyde organic substance in the presence of the dehydrogenase, the glycine and the aldehyde organic substance are converted into a β-hydroxy-α-amino acid under the co-catalysis of the D-aldolase and the racemase, and the β-hydroxy-α-amino acid generates the keto acid under the catalysis of the second dehydratase.

Further, the L-aldolase gene is selected from one or more of ltaE, ltaE_Pp, psald, dhaa, CC_3093, fbaA, itaA, glyA, or URA1, and is preferably ltaE_Pp or ltaE, wherein more preferably, the nucleotide sequence of the ltaE_Pp or ltaE gene is set forth in SEQ ID NO. 1 or SEQ ID NO. 2; the first dehydratase gene is selected from one or more of ilvA, tdcB, TDH, CHA1, TD2, A8H32_14290, Saut_1089, and C0627_08730, and is preferably ilvA or A8H32_14290, wherein more preferably, the nucleotide sequence of the ilvA or A8H32_14290 gene is set forth in SEQ ID NO. 3 or SEQ ID NO. 4; the dehydrogenase gene comprises one or more of adhE, adh, ADH7, xylB, adhA, xylW, ped, leuB, BADH, aldh, ACIAD1578, and qbdA, and is preferably xylB, wherein more preferably, the nucleotide sequence of the xylB gene is set forth in SEQ ID NO. 6. Further, the D-aldolase gene is selected from one or more of A0A1C9ZZ39_CHLRE, tasS, dna, cghG, folB, guaB, dus, dhaa, bhcC, NCTC12151_01614, A4G23_03658, OJAG_33340, and GGC03_18995, and is preferably A0A1C9ZZ39_CHLRE, wherein more preferably, the nucleotide sequence of the A0A1C9ZZ39_CHLRE gene is set forth in SEQ ID NO. 7; the racemase gene is selected from one or more of ILE2E_LENBU, agiA, puuE, PS659_05479, HRbin10_02390, CVS47_02795, HRbin08_01795, and MJ8_44540, and is preferably ILE2E_LENBU, wherein more preferably, the nucleotide sequence of the ILE2E_LENBU gene is set forth in SEQ ID NO. 8; the second dehydratase gene is selected from one or more of ilvA, tdcB, TDH, CHA1, TD2, A8H32_14290, Saut _1089, and C0627_08730, wherein more preferably, the nucleotide sequence of the ilvA gene is set forth in SEQ ID NO. 3.

Further, the first recombinant microorganism further comprises an enamine/imine deaminase gene, and is preferably ridA, wherein more preferably, the nucleotide sequence of the ridA gene is set forth in SEQ ID NO: 5.

Further, the alcoholic organic substance is selected from one or more of benzyl alcohol, 4-imidazolemethanol, 2-(methylthio)ethanol, indole-3-methanol, 2-hydroxyethyl-methyl phosphinic acid, *p*-hydroxybenzyl alcohol, 3,4-dihydroxybenzyl alcohol, *p*-methylbenzyl alcohol, phenethyl alcohol, *tert*-amyl alcohol, isobutanol, and ethanol.

In a second aspect, provided is a method for preparing a keto acid, wherein an enzymatic reaction is performed using glycine and an aldehyde organic substance as substrates, wherein during the enzymatic reaction process, the glycine and the aldehyde organic substance are converted into a β-hydroxy-α-amino acid, and then the β-hydroxy-α-amino acid is converted into the keto acid.

Preferably, the keto acid prepared comprises, but is not limited to, a keto acid having the following general formula: 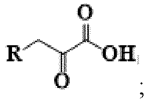 wherein the structural formula of R may be 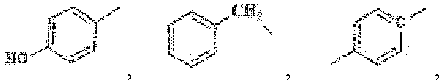 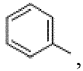 (CH₃)₂CH₂-, (CH₃)C-, CH₃-, 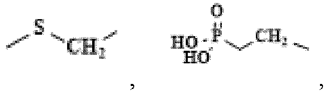 etc.

Further, an enzymatic reaction is performed using glycine and an aldehyde organic substance as substrates and using an enzyme produced by overexpression of a first recombinant microorganism comprising L-aldolase and first dehydratase genes as a catalyst, wherein the glycine and the aldehyde organic substance are converted into a β-hydroxy-α-amino acid under the independent catalysis of the L-aldolase, and the β-hydroxy-α-amino acid generates the keto acid under the catalysis of the first dehydratase;
or an enzymatic reaction is performed using glycine and an aldehyde organic substance as substrates and using an enzyme produced by overexpression of the third recombinant microorganism comprising a D-aldolase gene, a racemase gene, and a second dehydratase gene as a catalyst, wherein the glycine and the aldehyde organic substance are converted into a β-hydroxy-α-amino acid under the co-catalysis of the D-aldolase and the racemase, and the β-hydroxy-α-amino acid generates the keto acid under the catalysis of the second dehydratase.

Further, the L-aldolase gene is selected from one or more of ltaE, ltaE_Pp, psald, dhaa, CC_3093, fbaA, itaA, glyA, or URA1, and is preferably ltaE_Pp or ltaE, wherein more preferably, the nucleotide sequence of the ltaE_Pp or ltaE gene is set forth in SEQ ID NO. 1 or SEQ ID NO. 2; the first dehydratase gene is selected from one or more of ilvA, tdcB, TDH, CHA1, TD2, A8H32_14290, Saut _1089, and C0627_08730, and is preferably ilvA or A8H32_14290, wherein more preferably, the nucleotide sequence of the ilvA or A8H32_14290 gene is set forth in SEQ ID NO. 3 or SEQ ID NO. 4.

Further, the D-aldolase gene is selected from one or more of A0A1C9ZZ39_CHLRE, tasS, dna, cghG, folB, guaB, dus, dhaa, bhcC, NCTC12151_01614, A4G23_03658, OJAG_33340, and GGC03_18995, and is preferably A0A1C9ZZ39_CHLRE, wherein more preferably, the nucleotide sequence of the A0A1C9ZZ39_CHLRE gene is set forth in SEQ ID NO. 7; the racemase gene is selected from one or more of ILE2E_LENBU, agiA, puuE, PS659_05479, HRbin10_02390, CVS47_02795, HRbin08_01795, and MJ8_44540, and is preferably ILE2E_LENBU, wherein more preferably, the nucleotide sequence of the ILE2E_LENBU gene is set forth in SEQ ID NO. 8; the second dehydratase gene is selected from one or more of ilvA, tdcB, TDH, CHA1, TD2, A8H32_14290, Saut _1089, and C0627_08730, wherein more preferably, the nucleotide sequence of the ilvA gene is set forth in SEQ ID NO. 3.

Further, the construction of the first recombinant microorganism, the second recombinant microorganism, or the third recombinant microorganism by a genetic engineering method is included, and the genetic engineering method includes plasmid expression or genomic integration. Further, in a case that the construction is performed by means of plasmid expression, the plasmid vector used is selected from one or two of pZAlac and pZElac.

Further, the constructed recombinant microorganism is cultured and then subjected to an enzymatic reaction, wherein the culturing method for the recombinant microorganism is: inoculating the recombinant microorganism into a 2-xyT culture medium comprising ampicillin, kanamycin, and chloramphenicol, culturing at 20-60 °C for 3-6 h, adding IPTG to a final concentration of 0.3 mM, culturing for another 15-30 h and then centrifuging, and decanting the culture supernatant.

Further, during the enzymatic reaction process, the reaction temperature is 20-90 °C, and the pH of the reaction buffer is 6.5-8.5.

Further, the recombinant microorganism comprises one or more of recombinant *Escherichia coli, Bacillus, Corynebacterium, Saccharomyces,* or *Streptomyces.*

Further, the recombinant microorganism is selected from one or more of recombinant *Escherichia coli, Bacillus subtilis, Pseudomonas sp., Bacillus megaterium, Bacillus amyloliquefaciens, Corynebacterium glutamicum, Saccharomyces cerevisiae, Candida utilis,* or *Pichia pastoris.*

In a third aspect, provided is a recombinant microorganism for preparing a keto acid, wherein the recombinant microorganism is a first recombinant microorganism comprising L-aldolase and first dehydratase genes, and a second recombinant microorganism comprising a dehydrogenase; or the recombinant microorganism is a third recombinant microorganism comprising a D-aldolase gene, a racemase gene, and a second dehydratase gene, and a second recombinant microorganism comprising a dehydrogenase.

Preferably, the L-aldolase gene is selected from one or more of ltaE, ltaE_Pp, psald, dhaa, CC_3093, fbaA, itaA, glyA, or URA1, and is preferably ltaE_Pp or ltaE, wherein more preferably, the nucleotide sequence of the ltaE_Pp or ltaE gene is set forth in SEQ ID NO. 1 or SEQ ID NO. 2; the first dehydratase gene is selected from one or more of ilvA, tdcB, TDH, CHA1, TD2, A8H32_14290, Saut _1089, and C0627_08730, and is preferably ilvA or A8H32_14290, wherein more preferably, the nucleotide sequence of the ilvA or A8H32_14290 gene is set forth in SEQ ID NO. 3 or SEQ ID NO. 4; the dehydrogenase gene is selected from one or more of adhE, adh, ADH7, xylB, adhA, xylW, ped, leuB, BADH, aldh, ACIAD1578, and qbdA, and is preferably xylB, wherein more preferably, the nucleotide sequence of the xylB gene is set forth in SEQ ID NO. 6.

Alternatively, the D-aldolase gene is selected from one or more of A0A1C9ZZ39_CHLRE, tasS, dna, cghG, folB, guaB, dus, dhaa, bhcC, NCTC12151_01614, A4G23_03658, OJAG_33340, and GGC03_18995, and is preferably A0A1C9ZZ39_CHLRE, wherein more preferably, the nucleotide sequence of the A0A1C9ZZ39_CHLRE gene is set forth in SEQ ID NO. 7; the racemase gene is selected from one or more of ILE2E_LENBU, agiA, puuE, PS659_05479, HRbin10_02390, CVS47_02795, HRbin08_01795, and MJ8_44540, and is preferably ILE2E_LENBU, wherein more preferably, the nucleotide sequence of the ILE2E_LENBU gene is set forth in SEQ ID NO. 8; the second dehydratase gene is selected from one or more of ilvA, tdcB, TDH, CHA1, TD2, A8H32_14290, Saut _1089, and C0627_08730, wherein more preferably, the nucleotide sequence of the ilvA gene is set forth in SEQ ID NO. 3.

Further preferably, the first recombinant microorganism further comprises an enamine/imine deaminase gene, and is preferably ridA, wherein more preferably, the nucleotide sequence of the ridA gene is set forth in SEQ ID NO: 5.

In a fourth aspect, provided is a recombinant microorganism for preparing a keto acid, wherein the recombinant microorganism is a first recombinant microorganism comprising L-aldolase and first dehydratase genes; or the recombinant microorganism is a third recombinant microorganism comprising a D-aldolase gene, a racemase gene, and a second dehydratase gene;
preferably, the L-aldolase gene is selected from one or more of ltaE, ltaE_Pp, psald, dhaa, CC_3093, fbaA, itaA, glyA, or URA1, and is preferably ltaE_Pp or ltaE, wherein more preferably, the nucleotide sequence of the ltaE_Pp or ltaE gene is set forth in SEQ ID NO. 1 or SEQ ID NO. 2; the first dehydratase gene is selected from one or more of ilvA, tdcB, TDH, CHA1, TD2, A8H32_14290, Saut _1089, and C0627_08730, and is preferably ilvA or A8H32_14290, wherein more preferably, the nucleotide sequence of the ilvA or A8H32_14290 gene is set forth in SEQ ID NO. 3 or SEQ ID NO. 4;
alternatively, the D-aldolase gene is selected from one or more of A0A1C9ZZ39_CHLRE, tasS, dna, cghG, folB, guaB, dus, dhaa, bhcC, NCTC12151_01614, A4G23_03658, OJAG_33340, and GGC03_18995, and is preferably A0A1C9ZZ39_CHLRE, wherein more preferably, the nucleotide sequence of the A0A1C9ZZ39_CHLRE gene is set forth in SEQ ID NO. 7; the racemase gene is selected from one or more of ILE2E_LENBU, agiA, puuE, PS659_05479, HRbin10_02390, CVS47_02795, HRbin08_01795, and MJ8_44540, and is preferably ILE2E_LENBU, wherein more preferably, the nucleotide sequence of the ILE2E_LENBU gene is set forth in SEQ ID NO. 8; the second dehydratase gene is selected from one or more of ilvA, tdcB, TDH, CHA1, TD2, A8H32_14290, Saut _1089, and C0627_08730, wherein more preferably, the nucleotide sequence of the ilvA gene is set forth in SEQ ID NO. 3.

Further, the keto acid comprises, but is not limited to, a keto acid having the following general formula: 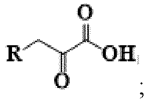 wherein the structural formula of R may be 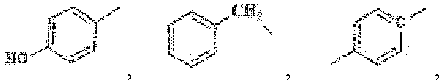 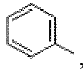 (CH₃)₂CH₂-, (CH₃)C-, CH₃-, 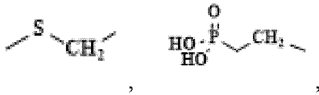 etc.

Further, the recombinant microorganism is selected from one or more of recombinant *Escherichia coli, Bacillus subtilis, Pseudomonas sp., Bacillus megaterium, Bacillus amyloliquefaciens, Corynebacterium glutamicum, Saccharomyces cerevisiae, Candida utilis,* or *Pichia pastoris.*

In a fifth aspect, provided is a recombinant DNA or biomaterial for use in preparing a keto acid, wherein the recombinant DNA or biomaterial
comprises L-aldolase and first dehydratase genes, and comprises a dehydrogenase gene; or the recombinant DNA or biomaterial comprises a D-aldolase gene, a racemase gene, and a second dehydratase gene, and comprises a dehydrogenase;
preferably, the L-aldolase gene is selected from one or more of ltaE, ltaE_Pp, psald, dhaa, CC_3093, fbaA, itaA, glyA, or URA1, and is preferably ltaE_Pp or ltaE, wherein more preferably, the nucleotide sequence of the ltaE_Pp or ltaE gene is set forth in SEQ ID NO. 1 or SEQ ID NO. 2; the first dehydratase gene is selected from one or more of ilvA, tdcB, TDH, CHA1, TD2, A8H32_14290, Saut _1089, and C0627_08730, and is preferably ilvA or A8H32_14290, wherein more preferably, the nucleotide sequence of the ilvA or A8H32_14290 gene is set forth in SEQ ID NO. 3 or SEQ ID NO. 4; the dehydrogenase gene is selected from one or more of adhE, adh, ADH7, xylB, adhA, xylW, ped, leuB, BADH, aldh, ACIAD1578, and qbdA, and is preferably xylB, wherein more preferably, the nucleotide sequence of the xylB gene is set forth in SEQ ID NO. 6;
alternatively, the D-aldolase gene is selected from one or more of A0A1C9ZZ39_CHLRE, tasS, dna, cghG, folB, guaB, dus, dhaa, bhcC, NCTC12151_01614, A4G23_03658, OJAG_33340, and GGC03_18995, and is preferably A0A1C9ZZ39_CHLRE, wherein more preferably, the nucleotide sequence of the A0A1C9ZZ39_CHLRE gene is set forth in SEQ ID NO. 7; the racemase gene is selected from one or more of ILE2E_LENBU, agiA, puuE, PS659_05479, HRbin10_02390, CVS47_02795, HRbin08_01795, and MJ8_44540, and is preferably ILE2E_LENBU, wherein more preferably, the nucleotide sequence of the ILE2E_LENBU gene is set forth in SEQ ID NO. 8; the second dehydratase gene is selected from one or more of ilvA, tdcB, TDH, CHA1, TD2, A8H32_14290, Saut _1089, and C0627_08730, wherein more preferably, the nucleotide sequence of the ilvA gene is set forth in SEQ ID NO. 3;
further preferably, the recombinant DNA or biomaterial further comprises an enamine/imine deaminase gene, and is preferably ridA, wherein more preferably, the nucleotide sequence of the ridA gene is set forth in SEQ ID NO: 5;
preferably, the biomaterial is an expression cassette, a transposon, a plasmid vector, a phage vector, or a virus vector.

In a sixth aspect, provided is a recombinant DNA or biomaterial for use in preparing a keto acid, wherein the recombinant DNA or biomaterialcomprises L-aldolase and first dehydratase genes; or the recombinant DNA or biomaterial comprises a D-aldolase gene, a racemase gene, and a second dehydratase gene;
preferably, the L-aldolase gene is selected from one or more of ltaE, ltaE_Pp, psald, dhaa, CC_3093, fbaA, itaA, glyA, or URA1, and is preferably ltaE_Pp or ltaE, wherein more preferably, the nucleotide sequence of the ltaE_Pp or ltaE gene is set forth in SEQ ID NO. 1 or SEQ ID NO. 2; the first dehydratase gene is selected from one or more of ilvA, tdcB, TDH, CHA1, TD2, A8H32_14290, Saut_1089, and C0627_08730, and is preferably ilvA or A8H32_14290, wherein more preferably, the nucleotide sequence of the ilvA or A8H32_14290 gene is set forth in SEQ ID NO. 3 or SEQ ID NO. 4;
alternatively, the D-aldolase gene is selected from one or more of A0A1C9ZZ39_CHLRE, tasS, dna, cghG, folB, guaB, dus, dhaa, bhcC, NCTC12151_01614, A4G23_03658, OJAG_33340, and GGC03_18995, and is preferably A0A1C9ZZ39_CHLRE, wherein more preferably, the nucleotide sequence of the A0A1C9ZZ39_CHLRE gene is set forth in SEQ ID NO. 7; the racemase gene is selected from one or more of ILE2E_LENBU, agiA, puuE, PS659_05479, HRbin10_02390, CVS47_02795, HRbin08_01795, and MJ8_44540, and is preferably ILE2E_LENBU, wherein more preferably, the nucleotide sequence of the ILE2E_LENBU gene is set forth in SEQ ID NO. 8; the second dehydratase gene is selected from one or more of ilvA, tdcB, TDH, CHA1, TD2, A8H32_14290, Saut _1089, and C0627_08730, wherein more preferably, the nucleotide sequence of the ilvA gene is set forth in SEQ ID NO. 3.

Preferably, the biomaterial is an expression cassette, a transposon, a plasmid vector, a phage vector, or a virus vector.

Further, the keto acid comprises, but is not limited to, a keto acid having the following general formula: 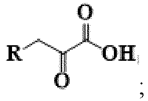 wherein the structural formula of R may be 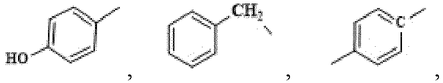 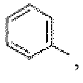 (CH₃)₂CH₂-, (CH₃)C-, CH₃-, 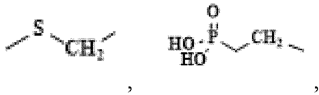 etc.

In a seventh aspect, provided is use of the recombinant microorganism for preparing a keto acid according to the third or fourth aspect, and the recombinant DNA or biomaterial for preparing a keto acid according to the fifth or sixth aspect for manufacturing a keto acid.

In an eighth aspect, provided is use of the recombinant microorganism for preparing a keto acid according to the third or fourth aspect, and the recombinant DNA or biomaterial for preparing a keto acid according to the fifth or sixth aspect for manufacturing an amino acid or an amino acid derivative.

In a ninth aspect, provided is a method for preparing a glycol organic substance, wherein the glycol organic substance is generated using the β-hydroxy-α-amino acid obtained by the method according to the first aspect in the presence of a deaminase, a decarboxylase, and a reductase. Beneficial Effects:
According to the method for preparing a keto acid described herein, by providing a novel multi-enzyme catalytic system, an enzymatic reaction is performed using glycine and different kinds of aldehydes in the presence of the multi-enzyme catalytic system. The number of the enzymes used herein is greatly less than that of a natural synthetic route, and the production cost is low. In addition, an artificial metabolic platform for keto acids is constructed, such that a plurality of important keto acids can be produced, e.g., phenylpyruvic acid, 4-methyl-2-oxopentanoic acid, pyruvic acid, and 2-oxobutyric acid, with simple operation, high yield, high substrate utilization rate, and low pollution.

The method for preparing a keto acid described herein is applied to the preparation process of amino acids and amino acid derivatives, achieving the preparation of a plurality of amino acids and amino acid derivatives with high output and high yield, and achieving industrial mass production.

The present disclosure is also applicable to the preparation of glycol organic substances.

### BRIEF DESCRIPTION OF THE DRAWINGS

FIG. 1 shows a graph of the process for producing phenylpyruvic acid from benzaldehyde and glycine in the R2 system.
FIG. 2 shows a graph of the process for producing 4-methyl-2-oxopentanoic acid from isobutyraldehyde and glycine in the R3 and R4 systems.
FIG. 3 shows a graph of the process for producing 2-oxobutyric acid from acetaldehyde and glycine in the R5 system.

### DETAILED DESCRIPTION

In order to make the technical means, creation characteristics, achieved purposes, and effects of the present disclosure easy to understand, the present disclosure is further illustrated below with reference to specific examples.

In the present disclosure, unless otherwise indicated, the scientific and technical terms used herein have the same meaning as commonly understood by those skilled in the art. In addition, the terms and laboratory procedures related to nucleic acid chemistry, molecular biology, cell and tissue culture, microbiology, and immunology used herein are the terms and conventional procedures widely used in the corresponding fields. Also, in order to better understand the present disclosure, the definitions and interpretations of the related terms are provided below.

It should be appreciated that the terms used herein are for the purpose of illustrating particular embodiments only, and are not intended to be limiting.

The articles "a", "an" and "the" are used herein to refer to one or more than one of the grammatical object of the article.

The use of alternatives (e.g., "or") should be understood to refer to one, two, or any combination of the alternatives. The term "and/or" should be interpreted as referring to one or both of the alternatives.

As used herein, the term "gene synthesis" refers to a generation process using recombinant DNA techniques or a production process using DNA or amino acid sequence synthesis techniques available and well known in the art.

The term "encode" or "code" refers to the inherent property of specific sequences of nucleotides in a polynucleotide, such as a gene, a cDNA, or an mRNA, to serve as a template in synthesis of other polymers and macromolecules in biological processes having either a defined sequence of nucleotides (i.e., rRNA, tRNA and mRNA) or a defined sequence of amino acids and the biological properties resulting therefrom. Thus, a gene encodes a protein if transcription and translation of an mRNA corresponding to the gene produces the protein in a cell or other biological system. Both the coding strand, which comprises a nucleotide sequence equivalent to the mRNA sequence and is usually provided in a sequence listing, and the non-coding strand, which is used as a template for transcription of a gene or cDNA, may be referred to as encoding the protein or other product of that gene or cDNA.

As used herein, the term "endogenous" refers to any substance derived from or produced within an organism, cell, tissue or system.

As used herein, the term "exogenous" refers to any substance introduced into or produced outside of an organism, cell, tissue or system.

As used herein, the term "expression" is defined as the transcription and/or translation of a particular nucleotide sequence driven by its promoter.

Unless otherwise specified, the "polynucleotide sequence encoding an amino acid sequence" includes all nucleotide sequences that are degenerate versions of each other and encode the same amino acid sequence. The phrase "nucleotide sequence encoding a protein or an RNA" may also include introns to the extent that the nucleotide sequence encoding the protein may contain an intron(s) in some versions.

As used herein, the term "vector" refers to a composition of matter that comprises an isolated nucleic acid and can be used to deliver the isolated nucleic acid to the interior of a cell. The transferred nucleic acid is typically ligated to, e.g., inserted into, a vector nucleic acid molecule. A vector may comprise sequences that direct autonomous replication in the cell, or may comprise sequences sufficient to allow integration into the host cell DNA. Many vectors are known in the art, including but not limited to plasmids, phagemids, artificial chromosomes, bacteriophages and animal viruses. Therefore, the term "vector" encompasses an autonomously replicating plasmid or virus.

In some embodiments, the L-aldolase-encoding gene comprises the ltaE gene, the fbaA gene of *Bacillus subtilis,* the psald gene of *Pseudomonas,* etc, and the dehydratase gene comprises the ilvA gene of *Escherichia coli,* the thadh gene of *Pseudomonas aeruginosa,* the A8H32_14290_14160 gene of *Burkholderia cepacia,* the thrc gene of *Bacillus subtilis,* etc.

A8H32_14290 denotes the dehydratase A8H32_14290_14160 from *Burkholderia cepacia.*

The gene may be wild-type or mutant gene. For example, in some embodiments, the A8H32_14290 gene may be wild-type A8H32_14290 or mutant A8H32_14290m, wherein the mutant A8H32_14290m refers to a A8H32_14290 mutant gene using PLP as a coenzyme, and the mutant A8H32_14290 is different from wild-type A8H32_14290 using PLP as a coenzyme. The mutant A8H32_14290 carries mutations S246A and E291A.

In some embodiments, the L-aldolase (ltaE), dehydratase (ilvA, tdcB), and enamine/imine deaminase (ridA) are from *Escherichia coli* genome.

In some embodiments, the L-aldolase (ltaE) may be from *Pseudomonas putida,* and the L-aldolase from *Pseudomonas putida* is denoted as ltaE-Pp herein.

In some embodiments, the dehydratase (A8H32_14290) may be from *Burkholderia cepacia.* The genes described above are obtained by PCR reaction using DNA primers.

The vector used herein comprises the polynucleotide molecule according to any one of the preceding items, wherein the vector further comprises common elements including, but not limited to, one or more of an origin of replication (ORI), an antibiotic resistance gene, a multiple cloning site (MCS), a promoter, an enhancer, a primer binding site, and a selectable marker.

In some embodiments, the vector is a plasmid vector, wherein the plasmid is one or two of pZAlac and pZElac.

A primer is designed based on the genomic sequence of *Pseudomonas putida* published by NCBI:

ltaE_Pp-
F:GAATTCATTAAAGAGGAGAAAGGTACCATGACAGACAAGAGCCAACAATTCGCCA
GCG(SEQ ID NO:10);

ltaE_Pp-
R:AGGTCGACATAGTTAATTTCTCCTACTAGTTCAGCCACCAATGATCGTGCGGATATC
CGC(SEQ ID NO:11).

A primer was designed based on the genomic sequence of *Escherichia coli* MG1655 published by NCBI:
ltaE-F:TTAAAGAGGAGAAAGGTACCATGATTGATTTACGCAGTGATACCG(SEQ ID NO: 12);
ltaE-R:CAGCCATAGTTAATTTCTCCTACTAGTTCAGCCACCAATGATCGTGCGGATATCC(SE Q ID NO: 13);
ilvA-F:GCGCGTTAAACTAGTAGGAGAAATTAACTATGGCTGACTCGCAACCCCTGTCC) (SEQ ID NO:14);
ilvA-R:CTTTCGTTTTATTTGATGCCTCTAGACTAACCCGCCAAAAAGAACCTGAACGCCG(S EQ ID NO:15).

A primer is designed based on the genomic sequence of *Bacillus* (*Bacillus badius*) published by NCBI:

pdh-
F:CATCCGCATTTAAGCTAGCAGGAGAAATTAACTATGAGCCTGGTGGAAAAACCA
GCAT(SEQ ID NO:16);

pdh-R:ATTTGATGCCTCTAGAGCTAGCTTAATTACGAATATCCCATTTCGGTTTAAC(SEQ ID NO: 17).

A primer is designed based on the genomic sequence of *Chlamydomonas reinhardtii* published by NCBI: A0A1C9ZZ39_CHLRE-F:GAATTCATTAAAGAGGAGAAAGGTACCATGCGCGCACTGGTTAGCAAAGCACG(S EQ ID NO:18); A0A1C9ZZ39_CHLRE-R:GCCCATAGTTAATTTCTCCTgctagcTCACTGGCCAGGACCACGACCACGAATC(SEQ ID NO:19).

A primer is designed based on the genomic sequence of *Lactobacillus buchneri* published by NCBI:

ILE2E_LENBU-
F:CCAGTGAGCTAGCAGGAGAAATTAACTATGGGCAAACTGGACAAAGCGAGCAAA
C(SEQ ID NO:20);

ILE2E_LENBU-
R:GACATAGTTAATTTCTCCTAAGCTTTCACCAGCCAATTTACCGGTGTCTTTCGGA(
SEQ ID NO:21).

Two groups of primers were designed based on the genomic sequence of *Burkholderia cepacia (Burkholderia thailandensis* E264) published by NCBI:

A8H32_14290-
F:GGTGGCTGAACTAGTAGGAGAAATTAACTATGTCGACCTCACCCCACCGCCCCGCT
CAT (SEQ ID NO:22);

A8H32_14290-
R:ATAGTTTTGCTCATAGTTAATTTCTCCTGCTAGCTCACGGCCACGACATGCGATGC
AGCCGAGC(SEQ ID NO:23);

A8H32_14290’-
F:TGGCTGGTAAAAGCTTAGGAGAAATTAACTATGTCGACCTCACCCCACCGCCCCG(
SEQ ID NO:24);

A8H32_14290’-
R:CGTTTTATTTGATGCCTCTAGATCACGGCCACGACATGCGATGCAGCCGAG(SEQ
ID NO:25).

A primer was designed based on the genomic sequence of *Escherichia coli* MG1655 published by NCBI:

ridA-F:
CATGTCGTGGCCGTGAGCTAGCAGGAGAAATTAACTATGAGCAAAACTATCGCGAC
GGAAAATGC(SEQ ID NO:26);

ridA-R:
GCCTTTCGTTTTATTTGATGCCTCTAGATTAGCGACGAACAGCGATCGCTTCGATC(S
EQ ID NO:27).

A primer is designed based on the genomic sequence of *Pseudomonas putida* published by NCBI:

xylB-
F:TCATTAAAGAGGAGAAAGGTACCATGGCGGTATTTGCCAGTGACTCTTTTG(SEQ
ID NO:28);

xylB-R:CATAGTTAATTTCTCCTGCTAGCTTAAATGCGGATGATGGTCGTCTTTG(SEQ
ID NO:29).

The corresponding gene fragments are obtained by PCR, digested by restriction endonuclease, and then ligated to plasmid pZElac through a ligase to give recombinant expression plasmid vectors pZE-ltaE _Pp-A8H32_14290, pZE-ltaE _Pp-A8H32_14290-ridA, pZE-ltaE-ilvA, pZE-ltaE-ilvA-ridA, pZA-xylB-pdh, and pZE-A0A1C9ZZ39_CHLRE-ILE2E_LENBU-A8H32_14290'.

Among them:
The nucleotide sequence of the ltaE_Pp gene is set forth in SEQ ID NO: 1.

The nucleotide sequence of the ltaE gene is set forth in SEQ ID NO: 2.

The nucleotide sequence of the ilvA gene is set forth in SEQ ID NO: 3.

The nucleotide sequence of the A8H32_14290 gene is set forth in SEQ ID NO: 4.

The nucleotide sequence of the ridA gene is set forth in SEQ ID NO: 5.

The nucleotide sequence of the xylB gene is set forth in SEQ ID NO: 6.

The nucleotide sequence of the A0A1C9ZZ39_CHLRE gene is set forth in SEQ ID NO: 7.

The nucleotide sequence of the ILE2E_LENBU gene is set forth in SEQ ID NO: 8.

The nucleotide sequence of the pdh gene used in Example 4 is set forth in SEQ ID NO: 9.

### Example 1

In this example, glycine and benzaldehyde were used as starting materials, and the product was phenylpyruvic acid.

The recombinant expression plasmid vectors pZE-ltaE_Pp- A8H32_14290 and pZE-ltaE_Pp-A8H32_14290-ridA were transformed into an expression strain *Escherichia coli* BL21 separately to give recombinant *Escherichia coli* marked as R1 and R2. The recombinant *Escherichia coli* was inoculated into a 2-xyT culture medium containing ampicillin and chloramphenicol, and the mixture was cultured in a 50 mL Erlenmeyer flask (liquid volume 10 mL) at 30 °C with a rotation speed of 240 rpm for 3-6 h. IPTG was added to a final concentration of 0.3 mM, and the mixture was cultured for another 20 h to enable protein expression. The mixture was centrifuged at 4 °C with a rotation speed of 8000 rpm for 5 min, and the supernatant culture medium was decanted to give a bacterial solution. The bacterial solution was subjected to an ice bath for further use.

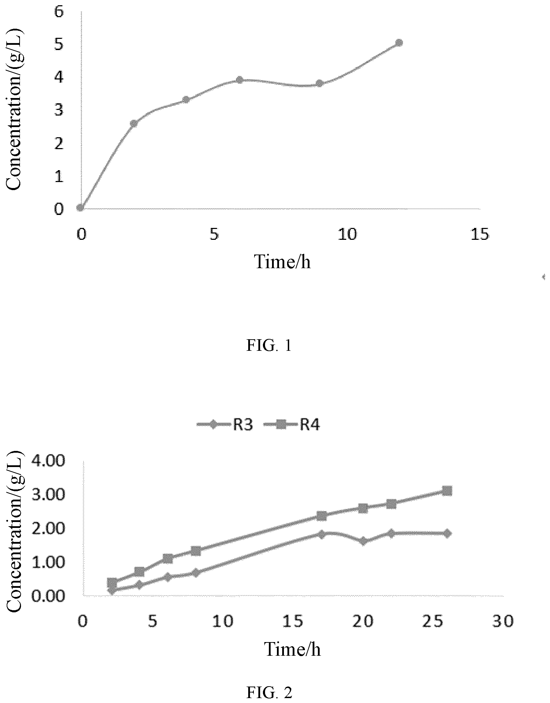

The bacterial solution described above was added to a 2 mL system containing 50 mM Tris-HCl buffer at pH 7.5. Glycine was added at a ratio of 20 g/L (the ratio of glycine to the system), and benzaldehyde (4.6 g/L) was added. The mixture was allowed to react under shaking in a shaker at 30 °C and 240 rpm for 24 h to give a transformation solution containing phenylpyruvic acid. Samples were taken every 2 h of catalysis to detect the generation of phenylpyruvic acid. The obtained transformation solution was diluted 20-fold with deionized water, and centrifuged at 12500 rpm/min for 10-15 min. The supernatant was pipetted and diluted, and high performance liquid chromatography analysis was performed. The concentrations of the substrates and product in the catalytic system were determined. According to the determination, 3.6 g/L phenylpyruvic acid was produced in the R1 system after 24 h of catalytic reaction, and 5 g/L phenylpyruvic acid was produced in the R2 system after 12 h of catalytic reaction. In the R2 system, the addition of the ridA gene could accelerate the conversion from the intermediate enamine to the final product, thus making the reaction faster and increasing the concentration and yield of the product (as shown in FIG. 1).

### Example 2

In this example, glycine and isobutyraldehyde were used as starting materials, and the product was 4-methyl-2-oxopentanoic acid.

The recombinant expression plasmid vectors pZE-ltaE-ilvA and pZE-ltaE-ilvA--ridA were transformed into an expression strain *Escherichia coli* BL21 separately to give recombinant *Escherichia coli* marked as R3 and R4. The recombinant *Escherichia coli* was inoculated into a 2-xyT culture medium containing ampicillin and chloramphenicol, and the mixture was cultured in a 50 mL Erlenmeyer flask (liquid volume 10 mL) at 30 °C with a rotation speed of 240 rpm for 3-6 h. IPTG was added to a final concentration of 0.3 mM, and the mixture was cultured for another 20 h. Then, the mixture was centrifuged at 4 °C with a rotation speed of 8000 rpm for 5 min, and the supernatant culture medium was decanted to give a bacterial solution. The bacterial solution was subjected to an ice bath for further use.

The bacterial solution described above was added to a 2 mL system containing 50 mM Tris-HCl buffer (pH 7.5), 20 g/L glycine, and 2.6 g/L isobutyraldehyde. The mixture was allowed to react under shaking in a shaker at 30 °C and 240 rpm for 24 h to give a transformation solution containing oxopentanoic acid. Samples were taken every 2 h of catalysis to detect the generation of oxopentanoic acid. The obtained transformation solution was diluted 20-fold with deionized water, and centrifuged at 12500 rpm/min for 10-15 min. The supernatant was pipetted and diluted, and high performance liquid chromatography analysis was performed. The concentration of the product in the catalytic system was determined. According to the determination, after 26 h of catalytic reaction, 1.9 g/L 4-methyl-2-oxopentanoic acid was produced in the R3 system, and 3.1 g/L 4-methyl-2-oxopentanoic acid was produced in the R4 system. The addition of the ridA gene could accelerate the conversion from the intermediate enamine to the final product, thus making the reaction faster and increasing the concentration and yield of the product (as shown in FIG. 2).

### Example 3

In this example, glycine and acetaldehyde were used as starting materials, and the product was 2-oxobutyric acid.

The recombinant expression plasmid vector pZE-ltaE-ilvA was transformed into an expression strain *Escherichia coli* BL21 to give recombinant *Escherichia coli* marked as R5. The recombinant *Escherichia coli* was inoculated into a 2-xyT culture medium containing ampicillin and chloramphenicol, and the mixture was cultured in a 50 mL Erlenmeyer flask (liquid volume 10 mL) at 30 °C with a rotation speed of 240 rpm for 3-6 h. IPTG was added to a final concentration of 0.3 mM, and the mixture was cultured for another 20 h. The mixture was centrifuged at 4 °C with a rotation speed of 8000 rpm for 5 min, and the supernatant culture medium was decanted to give a bacterial solution. The bacterial solution was subjected to an ice bath for further use.

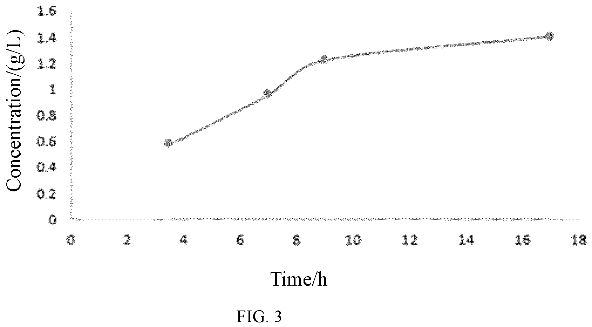

The bacterial solution described above was added to a 2 mL system containing 50 mM Tris-HCl buffer at pH 8.0, and 20 g/L glycine and 15 mL acetaldehyde were added. The mixture was fermented in a shaker at 30 °C and 240 rpm, and allowed to react under shaking for 3 h to give a transformation solution containing 2-oxobutyric acid. The obtained transformation solution was diluted 10-fold with deionized water, and centrifuged at 12500 rpm/min for 10-15 min. The supernatant was pipetted and diluted, and high performance liquid chromatography analysis was performed. The concentration of the product was determined. According to the determination, after 18 h of catalytic reaction, a total of 1.4 g/L 2-oxobutyric acid was produced (as shown in FIG. 3).

### Example 4

In this example, glycine and benzyl alcohol were used as starting materials, and the products were phenylpyruvic acid and phenylalanine.

The recombinant expression plasmid vectors pZE-ltaE_Pp-A8H32_14290 and pZA-xylB-pdh were electroporated into an expression strain *Escherichia coli* BL21 simultaneously to give recombinant *Escherichia coli* marked as R6. (The pdh gene encodes an enzyme which reduces phenylpyruvic acid to phenylalanine, and in this example, the substrate keto acid was further converted to an amino acid). The strain was inoculated into a 2-xyT culture medium containing ampicillin, kanamycin, and chloramphenicol, and the mixture was cultured in a 50 mL Erlenmeyer flask (liquid volume 10 mL) at 30 °C with a rotation speed of 240 rpm for 3-6 h. IPTG was added to a final concentration of 0.3 mM, and the mixture was cultured for another 20 h. The mixture was centrifuged at 4 °C with a rotation speed of 8000 rpm for 5 min, and the supernatant culture medium was decanted to give a bacterial solution. The bacterial solution was subjected to an ice bath for further use.

The bacterial solution described above was added to a 2 mL system containing 50 mM Tris-HCl buffer at pH 7.5. Glycine was added at a ratio of 20 g/L, and benzyl alcohol was added at a ratio of 4.6 g/L. The mixture was fermented in a shaker at 30 °C and 240 rpm, and allowed to react under shaking for 24 h to give a transformation solution containing phenylalanine. After 24 h of catalytic reaction, the obtained transformation solution was diluted 20-fold with deionized water, and centrifuged at 12500 rpm/min for 10-15 min. The supernatant was pipetted and diluted, and high performance liquid chromatography analysis was performed. The concentrations of the substrates and products in the catalytic system were determined. According to the determination, 2.3 g/L phenylpyruvic acid and 1.2 g/L phenylalanine were produced in the R6 system.

### Example 5

In this example, glycine and benzaldehyde were used as starting materials, and the product was phenylpyruvic acid (D-aldolase).

The recombinant expression plasmid vector pZE-A0A1C9ZZ39_CBLRE-ILE2E_LENBU-A8H32_14290' was transformed into an expression strain *Escherichia coli* BL21 to give recombinant *Escherichia coli* marked as R7. The recombinant *Escherichia coli* was inoculated into a 2-xyT culture medium containing ampicillin and chloramphenicol, and the mixture was cultured in a 50 mL Erlenmeyer flask (liquid volume 10 mL) at 30 °C with a rotation speed of 240 rpm for 3-6 h. IPTG was added to a final concentration of 0.3 mM, and the mixture was cultured for another 20 h to enable protein expression. The mixture was centrifuged at 4 °C with a rotation speed of 8000 rpm for 5 min, and the supernatant culture medium was decanted to give a bacterial solution. The bacterial solution was subjected to an ice bath for further use.

The bacterial solution described above was added to a 2 mL system containing 50 mM Tris-HCl buffer at pH 7.5. Glycine was added at a ratio of 20 g/L (the ratio of glycine to the system), and 4.6 g/L benzaldehyde was added. The mixture was allowed to react under shaking in a shaker at 30 °C and 240 rpm for 24 h to give a transformation solution containing phenylpyruvic acid. Samples were taken every 2 h of catalysis to detect the generation of phenylpyruvic acid. The obtained transformation solution was diluted 20-fold with deionized water, and centrifuged at 12500 rpm/min for 10-15 min. The supernatant was pipetted and diluted, and high performance liquid chromatography analysis was performed. The concentrations of the substrates and product in the catalytic system were determined. According to the determination, after 24 h of catalytic reaction, 2.6 g/L phenylpyruvic acid was produced in the R7 system.

The foregoing shows and describes the general principles, principal features, and advantages of the present disclosure. It should be understood by those skilled in the art that the present disclosure is not limited to the examples described above, and the examples described above and the description in the specification are merely illustration of the principles of the present disclosure. Various changes and modifications may be made without departing from the spirit and scope of the present disclosure, and such changes and modifications are within the protection scope of the present disclosure as claimed. The present disclosure claims a scope of protection as defined by the appended claims and the equivalents thereof.

SEQUENCE LISTING

<210> 1

<211> 1041

<212> DNA

<213> Pseudomonas putida

<400> 1

atgacagaca agagccaaca attcgccagc gacaactatt ccggtatctg ccccgaagcc 60

tgggtggcga tggagaaagc caaccgcggc cacgaccgcg cctacggcga cgaccagtgg 120

accgagcgtg catcggagta cttccgcaat ctgttcgaaa ccgactgcga ggtgttcttt 180

gccttcaacg gcaccgcggc caactccctg gccctggcat ccctgtgcca gagctatcac 240

agcgtgatct gctccgagac cgcccacgtc gaaaccgacg aatgcggtgc accggagttt 300

ttctccaacg gctccaagct gctgacggcg gccagcgtca acggcaagct gacgccacag 360

tcgatccgcg aagtggcgct caaacgccag gatatccact accccaagcc gcgcgtggtg 420

accattaccc aggccaccga agtgggcacg gtgtaccgcc ccgacgagct gaaggcgatc 480

agcgccacct gcaaggagct gggcctgaac ctgcacatgg acggcgcacg ctttaccaat 540

gcctgtgcgt tcctgggctg cagcccggcc gaactgacct ggaaggccgg tgtggatgtg 600

ctgtgctttg gcggcaccaa gaacggcatg gcggtgggcg aagcgattct gttcttcaac 660

cgccagctgg ccgaagactt cgattatcgc tgcaagcagg ccgggcaact ggcgtcgaag 720

atgcgcttct tgtcggcgcc ctgggtcggg ctgctggaag atggcgcatg gttacgccat 780

ggcaaccacg ccaatcattg cgcgcaactg ctggcatcgc tggtcagtga cctgccgggg 840

gtagagctga tgttcccggt ggaggccaac ggggtgttcc tgcagatgcc agagcacgcc 900
atcgaggcgc tgcggggcaa gggctggcgc ttctatacct ttatcggcag cggtggcgcg 960
cgcttcatgt gctcgtggga taccgaagaa gcgcgggtgc gtgagctggc ggcggatatc 1020
cgcacgatca ttggtggctg a 1041

<210> 2
<211> 1002
<212> DNA
<213> Escherichia coli MG1655
<400> 2
atgattgatt tacgcagtga taccgttacc cgaccaagcc gcgccatgct cgaagcgatg 60
atggccgccc cggttgggga cgacgtttac ggagacgacc ctaccgttaa tgctctgcag 120
gactacgcag cagagctttc cggtaaagaa gccgccattt ttctgcctac cggcactcag 180
gccaacctgg tcgctctgct cagtcactgc gaacgcggcg aagagtatat tgtcggtcag 240
gccgcgcata actatctgtt tgaagccggt ggcgcggcgg tgctgggcag tattcaaccg 300
caacccatag acgcggctgc cgacggcacg ctaccgctgg ataaagtggc gatgaaaatc 360
aaacccgacg atatccattt cgcccgcacc aaattactca gtctggaaaa cacccacaac 420
ggcaaagtgt tgccgcggga atacctgaaa gaagcatggg aatttacccg cgagcgcaat 480
ctggcgctgc atgttgacgg tgcgcgcatc tttaatgccg tggtggctta cggctgcgaa 540
ctgaaagaga tcacgcaata ttgtgattcg ttcaccattt gcctgtcgaa aggtcttggg 600
acgccagtcg gttcattact cgtcggtaat cgtgattaca ttaaacgtgc cattcgctgg 660
cggaaaatga caggtggcgg gatgcgccag tccggcattc tggctgccgc cgggatatat 720
gccctgaaaa ataacgttgc gcgcttgcag gaagaccacg acaacgctgc ctggatggcg 780
gagcagctgc gtgaagcagg cgcggatgtg atgcgtcagg acaccaatat gctgtttgtt 840
cgcgtcgggg aagaaaatgc tgccgcgtta ggcgaataca tgaaagcgag aaacgtgctg 900
attaacgcct cgccgattgt ccgcctggtg acgcatcttg acgtctcgcg cgaacaactg 960
gcggaagtcg ccgcccactg gcgtgcattc ctggcgcgtt aa 1002

<210> 3

<211>  1545

<212>  DNA

<213>  Escherichia coli MG1655

<400>  3

atggctgact cgcaacccct gtccggtgct ccggaaggtg ccgaatattt aagagcagtg      60

ctgcgcgcgc cggtttacga ggcggcgcag gttacgccgc tacaaaaaat ggaaaaactg     120

tcgtcgcgtc ttgataacgt cattctggtg aagcgcgaag atcgccagcc agtgcacagc     180

tttaagctgc gcggcgcata cgccatgatg gcgggcctga cggaagaaca gaaagcgcac     240

ggcgtgatca ctgcttctgc gggtaaccac gcgcagggcg tcgcgttttc ttctgcgcgg     300

ttaggcgtga aggccctgat cgttatgcca accgccaccg ccgacatcaa agtcgacgcg     360

gtgcgcggct tcggcggcga agtgctgctc cacggcgcga actttgatga agcgaaagcc     420

aaagcgatcg aactgtcaca gcagcagggg ttcacctggg tgccgccgtt cgaccatccg     480

atggtgattg ccgggcaagg cacgctggcg ctggaactgc tccagcagga cgcccatctc     540

gaccgcgtat ttgtgccagt cggcggcggc ggtctggctg ctggcgtggc ggtgctgatc     600

aaacaactga tgccgcaaat caaagtgatc gccgtagaag cggaagactc cgcctgcctg     660

aaagcagcgc tggatgcggg tcatccggtt gatctgccgc gcgtagggct atttgctgaa     720

ggcgtagcgg taaaacgcat cggtgacgaa accttccgtt tatgccagga gtatctcgac     780

gacatcatca ccgtcgatag cgatgcgatc tgtgcggcga tgaaggattt attcgaagat     840

gtgcgcgcgg tggcggaacc ctctggcgcg ctggcgctgg cgggaatgaa aaaatatatc     900

gccctgcaca acattcgcgg cgaacggctg gcgcatattc tttccggtgc caacgtgaac     960

ttccacggcc tgcgctacgt ctcagaacgc tgcgaactgg gcgaacagcg tgaagcgttg    1020

ttggcggtga ccattccgga agaaaaaggc agcttcctca aattctgcca actgcttggc    1080

gggcgttcgg tcaccgagtt caactaccgt tttgccgatg ccaaaaacgc ctgcatcttt    1140

gtcggtgtgc gcctgagccg cggcctcgaa gagcgcaaag aaattttgca gatgctcaac    1200

gacggcggct acagcgtggt tgatctctcc gacgacgaaa tggcgaagct acacgtgcgc    1260

tatatggtcg gcggacgtcc atcgcatccg ttgcaggaac gcctctacag cttcgaattc    1320

ccggaatcac cgggcgcgct gctgcgcttc ctcaacacgc tgggtacgta ctggaacatt    1380

tctttgttcc actatcgcag ccatggcacc gactacgggc gcgtactggc ggcgttcgaa    1440

cttggcgacc atgaaccgga tttcgaaacc cggctgaatg agctgggcta cgattgccac    1500

gacgaaacca ataacccggc gttcaggttc tttttggcgg gttag                    1545

<210> 4

<211> 1032

<212> DNA

<213> Burkholderia cepacia (Burkholderia thailandensis E264)

<400> 4

atgtcgacct caccccaccg ccccgctcat ctgacgatca acggcgagcc gattccgacg 60

ctcgacgaaa tcgccgcgca gcacttcgcg ctcacgccgt gggtcacgcg tacgccggtg 120

ttcgagcgcg ccgatctgcc atcgctcgac aacacgcacg tgaacttcaa gttcgagctg 180

ctgcaggcga gcggcagctt caaggcgcgc ggcgcgttca cgaacctgct cgcgctcggc 240

gaggacagaa agcgcgcggg cgtcacctgc gtgtcggcgg gcaatcatgc ggcggccgtc 300

gcttatgcgg cgatgcggct cggcacgagc gcgaaggtcg tgatgttcga caccgcgagc 360

ccggcgcgcg tcgagctctg ccaccagtac cgcgcggagg tcgtgatggc gtcgagcccc 420

gccgacgcgt tcgagatcgt ccggcgaatc gaggccgagg aaggccgctt cttcgtgcat 480

ccgttcaacg gctatcacac gattctcggc accgcgacgc tcggctacga atgggcgacg 540

cagacgcccg acctcgatgc ggtgatcgtg ccgatcggcg gcggcggcct cgccgcgggc 600

acctcgaccg cgctgcggct tgcgaatccg cgcgtgcacg tgtacggcgt cgagccggaa 660

ggtgcggatg tgatgagccg cagcttcgcc gcgaatcgcc cggtccgcat gggcgcgatg 720

catacgatcg ccgattcgct gatggcgccg cacaccgagg agtacagcta cgagctgtgc 780

cgccgccatg tcgaccggat cgtcaccgtc tcggacgacg cgctgcgagc ggcgatgctc 840

tatttgttca cgcatctgaa agtggcggtc gagcccgcgt gcgcggccgc gacggccgcc 900

ctcctcgggc cgttgcgcga gtcgctgcaa ggcaagcgtg ttggcgtgct gctgtcgggc 960

accaacatcg attcgacggc gctcgccacg catctcgagc atgctcggct gcatcgcatg 1020

tcgtggccgt ga 1032

<210> 5

<211> 387

<212> DNA

<213> Escherichia coli

<400> 5

atgagcaaaa ctatcgcgac ggaaaatgca ccggcagcta tcggtcctta cgtacagggc 60

gttgatctgg gcaatatgat catcacctcc ggtcagatcc cggtaaatcc gaaaacgggc 120

gaagtaccgg cagacgtcgc tgcacaggca cgtcagtcgc tggataacgt aaaagcgatc 180

gtcgaagccg ctggcctgaa agtgggcgac atcgttaaaa ctaccgtgtt tgtaaaagat 240

ctgaacgact tcgcaaccgt aaacgccact tacgaagcct tcttcaccga acacaacgcc 300

accttcccgg cacgttcttg cgttgaagtt gcccgtctgc cgaaagacgt gaagattgag 360

atcgaagcga tcgctgttcg tcgctaa 387

<210> 6

<211> 1479

<212> DNA

<213> Pseudomonas putida

<400> 6

atggcggtat ttgccagtga ctcttttggc cagctgaaag tggagaaaat tatgactgcc 60

cagtggaacc actacattaa cggggaatac gtatcacccg aatctgaaga gtatatccac 120

gagttcatcc caaccacggc tttgccgggt gactcaatcg caaggggctc ggcagctgac 180

gttgataagg ctgttcgtgc cgcggcagcg gctcagcctg cctggaatgc acgcaagcca 240

attgagcggg gtcgtatcct tctcgccata gctcgtttgg ttcgcgccaa cgcagcggct 300

ttctgcgcca aagaagcgga agaaactggc aagcctctga agatggccgc ctttgagatc 360

gaggcatgtg ctcagtactt tgagtattac ggcggtttgg cgacagccat ccagggcgaa 420

accatcaacc tcggccccag ctaccacgcc tataccaccc gagagccatt tggagtggtg 480

ggggtcatcc tgccgtggaa ttcgccactg aaccaagctg ggcgagccat tgccccggca 540

ttggttagcg ggaacaccgt ggtggtcaaa ccttcagagt tcacctcggt gacgatgctc 600

cagttcgcgg aactggttgt gaaagaggca gggttgccac caggcgtatt gaacgtggtt 660

accggcaccg gtaaggaaac cggtgagcct ctggttaaac accctctgat ccgaaaggtt 720

gctttcaccg gttctgtccg tgccggacgg gagatcggca agctcgccgc agatcgcatc 780

attccgctgt cgctcgaatt gggcggcaaa tccccgaaca ttgtcttcga agacgcagat 840

ctggatcgag ctgtcgcggg tagcgtcttt gccttcaccg tcaacactgg tcaagtctgt 900

ctcgccggga cccgttgcct ggtgcatgag tcgatttttg aaaaattctc caagaagctt 960

gccggtgctg tagaggcgct tcagttcagc gacggcgaaa gcttcggtct cggcccccta 1020

acgaccaagg ctcagtttga gcaggttcat cgttacaacg agctggccat ccaggagggg 1080
gctcattgct tggtcggtgg ggaagctcca agtgacaaaa ccggctggta cgtacgaccc 1140
accgtctaca ccaacgtcaa caactcgatg cggattgctc gggaagaaat tttcggaccc 1200
gttctggtac tgattccgtt caaggacgaa aacgaggcgg tggccatcgc gaatgactcg 1260
gactacgggc tcgcggctgg cgtatggacc accgatctgg ctcgcgcgca ccgcgtatcc 1320
gctcaaatcg aagcgggcca ggtgtacgtc aacgaatatc catcaggtgg cgttgagact 1380
ccattcggcg gtttcaagca aagcggccat gggcgcgaga agggcattga agcactccac 1440
cattacaccc aaacaaagac gaccatcatc cgcatttaa 1479

<210> 7
<211> 1287
<212> DNA
<213> Chlamydomonas reinhardtii
<400> 7
atgcgcgcac tggttagcaa agcacgcctg gcacatagcg tgggtggtcg tgcgagccag 60
gcgacccgtt gcgcagcaac cattagcgca agccgtgcac cggcccatct gggtgatgcc 120
ctgcatgatg ttgatacccc ggcactgatt ctggatctgg atgcatttga tcgtaactgt 180
gaaaaactga aaggtgttat ggcaggtttt cctggtgttg cagttcgtcc gcatgcaaaa 240
gcacataaat gtgcagaagt tgcccgccgt cagctgcagc tgctgggtgc taaaggtgtt 300
tgctgtcaga aagttattga agcggaagca atggcagaag gtggtgtttc cgatctgctg 360
ctgagcaatg aagtgattgc acctcgtaaa atcgatcgtc tggttggtct ggcagcggcc 420
ggtgcacgcg ttggtgtgtg ttatgaacgt gaagataatc tgcgtcagct gaatgccgca 480
gcagcagcac gtggtacaca tctggatgtt ctggttgaac tgaatgttgg tcaggatcgt 540
tgtggtgtta atagcgccga tgaagttgtt cagctggccc gtgcagccgc cggtctggat 600
aatgttcgtt ttgcaggtat tcaggcatat catggtggtc tgcagcatgt tcgtgatccg 660
cgtgatcgcg cacagcgtgt tggtcaggtt gttggtcgtg cacgtgcagc agttgatgcg 720
ctgaaagcag caggtctgcc atgtgatacc gttacaggtg gtggtacagg tacatatcgt 780
gtggaagcag caagtggtgt ttttacggaa gttcagccgg gtagttttgc attttctgat 840
gccgattatg cgcgtaatct gcaggaagat ggtggtgtgg gtgaatggga acagagcctg 900
tgggttctga cccaggtgat gagcgtgacc ccggcacgtg gtctggcagt tgttgatgca 960

ggtacaaaag cagtgtctct ggatagtggt ccgccgcgtc tgccgccggc atttgaagct 1020
gcatacggta caatgatgga atatggctct ggtggtgatg aacatggtaa actgatgtgg 1080
ccgcagggtg cgtatcagct gccgatgtca ctgcctgaag ttggtagcct gctgctgctg 1140
cagccgggtc attgtgatcc gacagttaat ctgtatgatt ggctggtggc cgcgcgtcgt 1200
cagcagggtg gtcagcagca gggtggcgtt gatggttggc gtgttgaagc agtttggccg 1260
attcgtggtc gtggtcctgg ccagtga 1287

<210> 8
<211> 1353
<212> DNA
<213> Lactobacillus buchneri
<400> 8
atgggcaaac tggacaaagc gagcaaactg atcgacgaag aaaataaata ctacgcacgt 60
agcgcgcgta tcaactacta caacctggtg attgaccacg cccacggcgc aaccctggtg 120
gatgtggatg gaaataaata tatcgatctg ctggcaagcg caagcgcaat taatgttggt 180
catacccatg aaaaagttgt gaaagcaatt gcagatcaag cacaaaaact gattcattat 240
accccggcat acttccacca cgttccgggt atggaactga gcgaaaaact ggcaaaaatt 300
gcaccgggta atagccctaa aatggtgagt tttggaaata gcggaagcga tgcaaatgat 360
gcaattatca aatttgcacg tgcatatacg ggacgtcagt atattgttag ttacatgggt 420
agctatcacg gaagcaccta cggtagccag acactgtcag gttcaagcct gaatatgacc 480
cgcaaaatcg gtccgatgct gccgagcgtt gttcatgttc cgtatccgga ttcatatcgc 540
acctacccgg gagaaactga gcacgatgtt agcctgcgtt attttaatga gtttaaaaag 600
ccgttcgaga gctttctgcc tgcagatgag accgcatgtg ttctgattga accgattcag 660
ggcgatggag gtattattaa agcaccggaa gaatatatgc agctggtgta taaattttgt 720
catgaacatg gtatcctgtt tgcaattgat gaagttaatc aaggcctggg tcgtacagga 780
aaaatgtggg caatccagca gtttaaagat attgaaccgg atctgatgag cgttggtaaa 840
agcctggcgt ctggtatgcc gctgagcgca gttatcggta aaaaggaagt tatgcagagc 900
ctggacgcac ctgcacacct gtttaccacc gcaggtaatc cggtttgtag tgcagcaagc 960
ctggcaactc tggacgttat tgaatatgag gggctggtgg agaaaagcgc aaccgacggt 1020
gcatatgcca aacaacgttt tctggaaatg caacagcgcc atccgatgat cggagatgtt 1080

cgtatgtggg ggctgaatgg gggtattgaa ctggttaaag acccgaaaac caaagaacct 1140
gatagtgacg cagcaaccaa agtgatttac tacgcctttg cacacggggt tgtaattatt 1200
acgctggcag gcaacattct gcgctttcaa ccgccgctgg ttatcccgcg tgaacaactg 1260
gatcaggcac tgcaggtgct ggatgacgcc tttaccgcag tcgaaaatgg ggaagttacc 1320
attccgaaag acaccggtaa aattggctgg taa 1353

<210> 9
<211> 1143
<212> DNA
<213> Bacillus (Bacillus badius)
<400> 9
atgagcctgg tggaaaaaac cagcattatt aaagatttta ccctgtttga aaaaatgagc 60
gaacatgaac aggttgtttt ttgtaatgat ccggcaaccg gtctgcgtgc aattattgca 120
attcatgata ccaccctggg tccggcactg ggtggttgtc gtatgcagcc gtataatagc 180
gttgaagaag cactggaaga tgcactgcgt ctgagcaaag gtatgaccta taaatgtgca 240
gcaagcgatg ttgattttgg tggtggtaaa gcagttatta ttggtgatcc gcagaaagat 300
aaaagcccgg aactgtttcg tgcatttggt cagtttgttg atagcctggg tggtcgtttt 360
tataccggta ccgatatggg taccaatatg gaagatttta ttcatgcaat gaaagaaacc 420
aattgtattg ttggtgttcc ggaagcatat ggtggtggtg gtgatagcag cattccgacc 480
gcaatgggtg ttctgtatgg tattaaagca accaataaaa tgctgtttgg taaagatgat 540
ctgggtggtg ttacctatgc aattcagggt ctgggtaaag ttggttataa agttgcagaa 600
ggtctgctgg aagaaggtgc acatctgttt gttaccgata ttaatgaaca gaccctggaa 660
gcaattcagg aaaaagcaaa aaccaccagc ggtagcgtta ccgttgttgc aagcgatgaa 720
atttatagcc aggaagcaga tgtttttgtt ccgtgtgcat ttggtggtgt tgttaatgat 780
gaaaccatga aacagtttaa agttaaagca attgcaggta gcgcaaataa tcagctgctg 840
accgaagatc atggtcgtca tctggcagat aaaggtattc tgtatgcacc ggattatatt 900
gttaatagcg gtggtctgat tcaggttgca gatgaactgt atgaagttaa taaagaacgt 960
gttctggcaa aaaccaaaca tatttatgat gcaattctgg aagtttatca gcaggcagaa 1020
ctggatcaga ttaccaccat ggaagcagca aatcgtatgt gtgaacagcg tatggcagca 1080
cgtggtcgtc gtaatagctt ttttaccagc agcgttaaac cgaaatggga tattcgtaat 1140

taa 1143

SEQ ID NO:10

GAATTCATTAAAGAGGAGAAAGGTACCATGACAGACAAGAGCCAACAATTCGCCAG

CG

SEQ ID NO:11

AGGTCGACATAGTTAATTTCTCCTACTAGTTCAGCCACCAATGATCGTGCGGATATCCG

C

SEQ ID NO:12

TTAAAGAGGAGAAAGGTACCATGATTGATTTACGCAGTGATACCG

SEQ ID NO:13

CAGCCATAGTTAATTTCTCCTACTAGTTCAGCCACCAATGATCGTGCGGATATCC

SEQ ID NO:14

GCGCGTTAAACTAGTAGGAGAAATTAACTATGGCTGACTCGCAACCCCTGTCC

SEQ ID NO:15

CTTTCGTTTTATTTGATGCCTCTAGACTAACCCGCCAAAAAGAACCTGAACGCCG

SEQ ID NO:16

CATCCGCATTTAAGCTAGCAGGAGAAATTAACTATGAGCCTGGTGGAAAAAACCAG

CAT

SEQ ID NO:17

ATTTGATGCCTCTAGAGCTAGCTTAATTACGAATATCCCATTTCGGTTTAAC

SEQ ID NO:18

GAATTCATTAAAGAGGAGAAAGGTACCATGCGCGCACTGGTTAGCAAAGCACG

SEQ ID NO:19
GCCCATAGTTAATTTCTCCTgctagcTCACTGGCCAGGACCACGACCACGAATC

SEQ ID NO:20
CCAGTGAGCTAGCAGGAGAAATTAACTATGGGCAAACTGGACAAAGCGAGCAAAC

SEQ ID NO:21
GACATAGTTAATTTCTCCTAAGCTTTCACCAGCCAATTTTACCGGTGTCTTTCGGA

SEQ ID NO:22
GGTGGCTGAACTAGTAGGAGAAATTAACTATGTCGACCTCACCCCACCGCCCCGCTC
AT

SEQ ID NO:23
ATAGTTTTGCTCATAGTTAATTTCTCCTGCTAGCTCACGGCCACGACATGCGATGCA
GCCGAGC

SEQ ID NO:24
TGGCTGGTAAAAGCTTAGGAGAAATTAACTATGTCGACCTCACCCCACCGCCCCG

SEQ ID NO:25
CGTTTTATTTGATGCCTCTAGATCACGGCCACGACATGCGATGCAGCCGAG

SEQ ID NO:26
CATGTCGTGGCCGTGAGCTAGCAGGAGAAATTAACTATGAGCAAAACTATCGCGAC
GGAAAATGC

SEQ ID NO:27

GCCTTTCGTTTTATTTGATGCCTCTAGATTAGCGACGAACAGCGATCGCTTCGATC

SEQ ID NO:28

TCATTAAAGAGGAGAAAGGTACCATGGCGGTATTTGCCAGTGACTCTTTTG

SEQ ID NO:29

CATAGTTAATTTCTCCTGCTAGCTTAAATGCGGATGATGGTCGTCTTTG

## Claims

1. A method for preparing a keto acid, wherein an enzymatic reaction is performed using glycine and an alcoholic organic substance as substrates, wherein during the enzymatic reaction process, the alcoholic organic substance is converted into an aldehyde organic substance, the glycine and the aldehyde organic substance are converted into a β-hydroxy-α-amino acid, and then the β-hydroxy-α-amino acid is converted into the keto acid.

2. The method as claimed in claim 1, wherein the keto acid prepared comprises, but is not limited to, a keto acid having the following general formula: 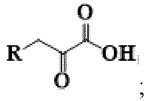 wherein the structural formula of R may be 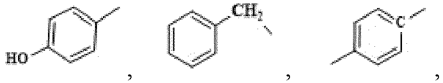 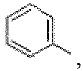 (CH₃)₂CH₂-, (CH₃)C-, CH₃-, 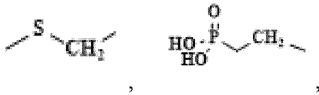 etc.

3. The method for preparing a keto acid as claimed in claim 1 or 2, wherein an enzymatic reaction is performed using glycine and an alcoholic organic substance as substrates and using an enzyme produced by overexpression of a first recombinant microorganism comprising L-aldolase and first dehydratase genes and a second recombinant microorganism comprising a dehydrogenase as a catalyst, wherein the alcoholic organic substance is converted into an aldehyde organic substance in the presence of the dehydrogenase, the glycine and the aldehyde organic substance are converted into a β-hydroxy-α-amino acid under the independent catalysis of the L-aldolase, and the β-hydroxy-α-amino acid generates the keto acid under the catalysis of the first dehydratase;
or, an enzymatic reaction is performed using an enzyme produced by overexpression of a third recombinant microorganism comprising a D-aldolase gene, a racemase gene, and a second dehydratase gene and a second recombinant microorganism comprising the dehydrogenase as a catalyst, wherein the alcoholic organic substance is converted into an aldehyde organic substance in the presence of the dehydrogenase, the glycine and the aldehyde organic substance are converted into a β-hydroxy-α-amino acid under the co-catalysis of the D-aldolase and the racemase, and the β-hydroxy-α-amino acid generates the keto acid under the catalysis of the second dehydratase.

4. The method for preparing a keto acid as claimed in claim 3, wherein the L-aldolase gene is selected from one or more of ltaE, ltaE_Pp, psald, dhaa, CC_3093, fbaA, itaA, glyA, or URA1, and is preferably ltaE_Pp or ltaE, wherein more preferably, the nucleotide sequence of the ltaE_Pp or ltaE gene is set forth in SEQ ID NO. 1 or SEQ ID NO. 2; the first dehydratase gene is selected from one or more of ilvA, tdcB, TDH, CHA1, TD2, A8H32_14290, Saut_1089, and C0627_08730, and is preferably ilvA or A8H32_14290, wherein more preferably, the nucleotide sequence of the ilvA or A8H32_14290 gene is set forth in SEQ ID NO. 3 or SEQ ID NO. 4; the dehydrogenase gene comprises one or more of adhE, adh, ADH7, xylB, adhA, xylW, ped, leuB, BADH, aldh, ACIAD1578, and qbdA, and is preferably xylB, wherein more preferably, the nucleotide sequence of the xylB gene is set forth in SEQ ID NO. 6.

5. The method for preparing a keto acid as claimed in claim 3, wherein the D-aldolase gene is selected from one or more of A0A1C9ZZ39_CHLRE, tasS, dna, cghG, folB, guaB, dus, dhaa, bhcC, NCTC12151_01614, A4G23_03658, OJAG_33340, and GGC03_18995, and is preferably A0A1C9ZZ39_CHLRE, wherein more preferably, the nucleotide sequence of the A0A1C9ZZ39_CHLRE gene is set forth in SEQ ID NO. 7; the racemase gene is selected from one or more of ILE2E_LENBU, agiA, puuE, PS659_05479, HRbin10_02390, CVS47_02795, HRbin08_01795, and MJ8_44540, and is preferably ILE2E_LENBU, wherein more preferably, the nucleotide sequence of the ILE2E_LENBU gene is set forth in SEQ ID NO. 8; the second dehydratase gene is selected from one or more of ilvA, tdcB, TDH, CHA1, TD2, A8H32_14290, Saut_1089, and C0627_08730, wherein more preferably, the nucleotide sequence of the ilvA gene is set forth in SEQ ID NO. 3.

6. The method for preparing a keto acid as claimed in any one of claims 1-5, wherein the first recombinant microorganism further comprises an enamine/imine deaminase gene, and is preferably ridA, wherein more preferably, the nucleotide sequence of the ridA gene is set forth in SEQ ID NO: 5.

7. The method for preparing a keto acid as claimed in any one of claims 1-6, wherein the alcoholic organic substance is selected from one or more of benzyl alcohol, 4-imidazolemethanol, 2-(methylthio)ethanol, indole-3-methanol, 2-hydroxyethyl-methyl phosphinic acid, *p-*hydroxybenzyl alcohol, 3,4-dihydroxybenzyl alcohol, *p*-methylbenzyl alcohol, phenethyl alcohol, *tert*-amyl alcohol, isobutanol, and ethanol.

8. A method for preparing a keto acid, wherein an enzymatic reaction is performed using glycine and an aldehyde organic substance as substrates, wherein during the enzymatic reaction process, the glycine and the aldehyde organic substance are converted into a β-hydroxy-α-amino acid, and then the β-hydroxy-α-amino acid is converted into the keto acid.

9. The method as claimed in claim 8, wherein the keto acid prepared comprises, but is not limited to, a keto acid having the following general formula: 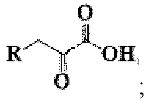 wherein the structural formula of R may be 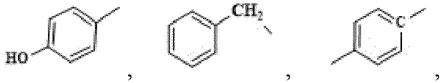 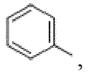 (CH₃)₂CH₂-, (CH₃)C-, CH₃-, 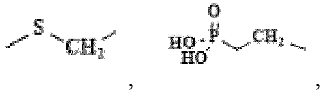 etc.

10. The method as claimed in claim 8 or 9, wherein an enzymatic reaction is performed using glycine and an aldehyde organic substance as substrates and using an enzyme produced by overexpression of a first recombinant microorganism comprising L-aldolase and first dehydratase genes as a catalyst, wherein the glycine and the aldehyde organic substance are converted into a β-hydroxy-α-amino acid under the independent catalysis of the L-aldolase, and the β-hydroxy-α-amino acid generates the keto acid under the catalysis of the first dehydratase;
or an enzymatic reaction is performed using glycine and an aldehyde organic substance as substrates and using an enzyme produced by overexpression of the third recombinant microorganism comprising a D-aldolase gene, a racemase gene, and a second dehydratase gene as a catalyst, wherein the glycine and the aldehyde organic substance are converted into a β-hydroxy-α-amino acid under the co-catalysis of the D-aldolase and the racemase, and the β-hydroxy-α-amino acid generates the keto acid under the catalysis of the second dehydratase.

11. The method as claimed in claim 10, wherein the L-aldolase gene is selected from one or more of ltaE, ltaE_Pp, psald, dhaa, CC_3093, fbaA, itaA, glyA, or URA1, and is preferably ltaE_Pp or ltaE, wherein more preferably, the nucleotide sequence of the ltaE_Pp or ltaE gene is set forth in SEQ ID NO. 1 or SEQ ID NO. 2;
the first dehydratase gene is selected from one or more of ilvA, tdcB, TDH, CHA1, TD2, A8H32_14290, Saut _1089, and C0627_08730, and is preferably ilvA or A8H32_14290, wherein more preferably, the nucleotide sequence of the ilvA or A8H32_14290 gene is set forth in SEQ ID NO. 3 or SEQ ID NO. 4.

12. The method as claimed in claim 10, wherein the D-aldolase gene is selected from one or more of A0A1C9ZZ39_CHLRE, tasS, dna, cghG, folB, guaB, dus, dhaa, bhcC, NCTC12151_01614, A4G23_03658, OJAG_33340, and GGC03_18995, and is preferably A0A1C9ZZ39_CHLRE, wherein more preferably, the nucleotide sequence of the A0A1C9ZZ39_CHLRE gene is set forth in SEQ ID NO. 7; the racemase gene is selected from one or more of ILE2E_LENBU, agiA, puuE, PS659_05479, HRbin10_02390, CVS47_02795, HRbin08_01795, and MJ8_44540, and is preferably ILE2E_LENBU, wherein more preferably, the nucleotide sequence of the ILE2E_LENBU gene is set forth in SEQ ID NO. 8; the second dehydratase gene is selected from one or more of ilvA, tdcB, TDH, CHA1, TD2, A8H32_14290, Saut _1089, and C0627_08730, wherein more preferably, the nucleotide sequence of the ilvA gene is set forth in SEQ ID NO. 3.

13. The method as claimed in claims 1-12, wherein the construction of the first recombinant microorganism, the second recombinant microorganism, or the third recombinant microorganism by a genetic engineering method is included, and the genetic engineering method includes plasmid expression or genomic integration.

14. The method as claimed in claim 13, wherein in a case that the construction is performed by means of plasmid expression, the plasmid vector used is selected from one or two of pZAlac and pZElac.

15. The method as claimed in claim 14, wherein the constructed recombinant microorganism is cultured and then subjected to an enzymatic reaction, wherein the culturing method for the recombinant microorganism is: inoculating the recombinant microorganism into a 2-xyT culture medium comprising ampicillin, kanamycin, and chloramphenicol, culturing at 20-60 °C for 3-6 h, adding IPTG to a final concentration of 0.3 mM, culturing for another 15-30 h and then centrifuging, and decanting the supernatant culture medium.

16. The method as claimed in claim 15, wherein during the enzymatic reaction process, the reaction temperature is 20-90 °C, and the pH of the reaction buffer is 6.5-8.5.

17. The preparation method for a keto acid as claimed in any one of claims 13-16, wherein the recombinant microorganism comprises one or more of recombinant *Escherichia coli, Bacillus, Corynebacterium, Saccharomyces,* or *Streptomyces.*

18. The method for preparing a keto acid as claimed in claim 17, wherein the recombinant microorganism is selected from one or more of recombinant *Escherichia coli, Bacillus subtilis, Pseudomonas sp., Bacillus megaterium, Bacillus amyloliquefaciens, Corynebacterium glutamicum, Saccharomyces cerevisiae, Candida utilis,* or *Pichia pastoris.*

19. A recombinant microorganism for preparing a keto acid, wherein the recombinant microorganism is a first recombinant microorganism comprising L-aldolase and first dehydratase genes, and a second recombinant microorganism comprising a dehydrogenase; or, the recombinant microorganism is a third recombinant microorganism comprising a D-aldolase gene, a racemase gene, and a second dehydratase gene, and a second recombinant microorganism comprising a dehydrogenase;
preferably, the L-aldolase gene is selected from one or more of ltaE, ltaE_Pp, psald, dhaa, CC_3093, fbaA, itaA, glyA, or URA1, and is preferably ltaE_Pp or ltaE, wherein more preferably, the nucleotide sequence of the ltaE_Pp or ltaE gene is set forth in SEQ ID NO. 1 or SEQ ID NO. 2; the first dehydratase gene is selected from one or more of ilvA, tdcB, TDH, CHA1, TD2, A8H32_14290, Saut _1089, and C0627_08730, and is preferably ilvA or A8H32_14290, wherein more preferably, the nucleotide sequence of the ilvA or A8H32_14290 gene is set forth in SEQ ID NO. 3 or SEQ ID NO. 4; the dehydrogenase gene is selected from one or more of adhE, adh, ADH7, xylB, adhA, xylW, ped, leuB, BADH, aldh, ACIAD1578, and qbdA, and is preferably xylB, wherein more preferably, the nucleotide sequence of the xylB gene is set forth in SEQ ID NO. 6;
alternatively, the D-aldolase gene is selected from one or more of A0A1C9ZZ39_CHLRE, tasS, dna, cghG, folB, guaB, dus, dhaa, bhcC, NCTC12151_01614, A4G23_03658, OJAG_33340, and GGC03_18995, and is preferably A0A1C9ZZ39_CHLRE, wherein more preferably, the nucleotide sequence of the A0A1C9ZZ39_CHLRE gene is set forth in SEQ ID NO. 7; the racemase gene is selected from one or more of ILE2E_LENBU, agiA, puuE, PS659_05479, HRbin10_02390, CVS47_02795, HRbin08_01795, and MJ8_44540, and is preferably ILE2E_LENBU, wherein more preferably, the nucleotide sequence of the ILE2E_LENBU gene is set forth in SEQ ID NO. 8; the second dehydratase gene is selected from one or more of ilvA, tdcB, TDH, CHA1, TD2, A8H32_14290, Saut _1089, and C0627_08730, wherein more preferably, the nucleotide sequence of the ilvA gene is set forth in SEQ ID NO. 3;
further preferably, the first recombinant microorganism further comprises an enamine/imine deaminase gene, and is preferably ridA, wherein more preferably, the nucleotide sequence of the ridA gene is set forth in SEQ ID NO: 5.

20. A recombinant microorganism for preparing a keto acid, wherein the recombinant microorganism is a first recombinant microorganism comprising L-aldolase and first dehydratase genes; or, the recombinant microorganism is a third recombinant microorganism comprising a D-aldolase gene, a racemase gene, and a second dehydratase gene;
preferably, the L-aldolase gene is selected from one or more of ltaE, ltaE_Pp, psald, dhaa, CC_3093, fbaA, itaA, glyA, or URA1, and is preferably ltaE_Pp or ltaE, wherein more preferably, the nucleotide sequence of the ltaE_Pp or ltaE gene is set forth in SEQ ID NO. 1 or SEQ ID NO. 2; the first dehydratase gene is selected from one or more of ilvA, tdcB, TDH, CHA1, TD2, A8H32_14290, Saut _1089, and C0627_08730, and is preferably ilvA or A8H32_14290, wherein more preferably, the nucleotide sequence of the ilvA or A8H32_14290 gene is set forth in SEQ ID NO. 3 or SEQ ID NO. 4;
alternatively, the D-aldolase gene is selected from one or more of A0A1C9ZZ39_CHLRE, tasS, dna, cghG, folB, guaB, dus, dhaa, bhcC, NCTC12151_01614, A4G23_03658, OJAG_33340, and GGC03_18995, and is preferably A0A1C9ZZ39_CHLRE, wherein more preferably, the nucleotide sequence of the A0A1C9ZZ39_CHLRE gene is set forth in SEQ ID NO. 7; the racemase gene is selected from one or more of ILE2E_LENBU, agiA, puuE, PS659_05479, HRbin10_02390, CVS47_02795, HRbin08_01795, and MJ8_44540, and is preferably ILE2E_LENBU, wherein more preferably, the nucleotide sequence of the ILE2E_LENBU gene is set forth in SEQ ID NO. 8; the second dehydratase gene is selected from one or more of ilvA, tdcB, TDH, CHA1, TD2, A8H32_14290, Saut _1089, and C0627_08730, wherein more preferably, the nucleotide sequence of the ilvA gene is set forth in SEQ ID NO. 3.

21. The recombinant microorganism as claimed in claim 19 or 20, wherein the keto acid comprises, but is not limited to, a keto acid having the following general formula: 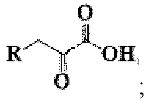 wherein the structural formula of R may be 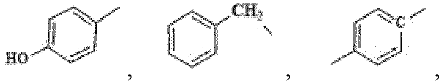 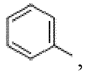 (CH₃)₂CH₂-, (CH₃)C-, CH₃-, 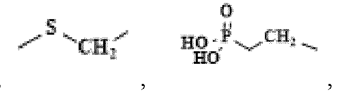 etc.

22. The recombinant microorganism as claimed in any one of claims 19-21, wherein the recombinant microorganism is selected from one or more of recombinant *Escherichia coli, Bacillus subtilis, Pseudomonas sp., Bacillus megaterium, Bacillus amyloliquefaciens, Corynebacterium glutamicum, Saccharomyces cerevisiae, Candida utilis,* or *Pichia pastoris.*

23. A recombinant DNA or biomaterial for use in preparing a keto acid, wherein the recombinant DNA or biomaterial
comprises L-aldolase and first dehydratase genes, and comprises a dehydrogenase gene; or, the recombinant DNA or biomaterial comprises a D-aldolase gene, a racemase gene, and a second dehydratase gene, and comprises a dehydrogenase;
preferably, the L-aldolase gene is selected from one or more of ltaE, ltaE_Pp, psald, dhaa, CC_3093, fbaA, itaA, glyA, or URA1, and is preferably ltaE_Pp or ltaE, wherein more preferably, the nucleotide sequence of the ltaE_Pp or ltaE gene is set forth in SEQ ID NO. 1 or SEQ ID NO. 2; the first dehydratase gene is selected from one or more of ilvA, tdcB, TDH, CHA1, TD2, A8H32_14290, Saut _1089, and C0627_08730, and is preferably ilvA or A8H32_14290, wherein more preferably, the nucleotide sequence of the ilvA or A8H32_14290 gene is set forth in SEQ ID NO. 3 or SEQ ID NO. 4; the dehydrogenase gene is selected from one or more of adhE, adh, ADH7, xylB, adhA, xylW, ped, leuB, BADH, aldh, ACIAD1578, and qbdA, and is preferably xylB, wherein more preferably, the nucleotide sequence of the xylB gene is set forth in SEQ ID NO. 6;
alternatively, the D-aldolase gene is selected from one or more of A0A1C9ZZ39_CHLRE, tasS, dna, cghG, folB, guaB, dus, dhaa, bhcC, NCTC12151_01614, A4G23_03658, OJAG_33340, and GGC03_18995, and is preferably A0A1C9ZZ39_CHLRE, wherein more preferably, the nucleotide sequence of the A0A1C9ZZ39_CHLRE gene is set forth in SEQ ID NO. 7; the racemase gene is selected from one or more of ILE2E_LENBU, agiA, puuE, PS659_05479, HRbin10_02390, CVS47_02795, HRbin08_01795, and MJ8_44540, and is preferably ILE2E_LENBU, wherein more preferably, the nucleotide sequence of the ILE2E_LENBU gene is set forth in SEQ ID NO. 8; the second dehydratase gene is selected from one or more of ilvA, tdcB, TDH, CHA1, TD2, A8H32_14290, Saut _1089, and C0627_08730, wherein more preferably, the nucleotide sequence of the ilvA gene is set forth in SEQ ID NO. 3;
further preferably, the recombinant DNA or biomaterial further comprises an enamine/imine deaminase gene, and is preferably ridA, wherein more preferably, the nucleotide sequence of the ridA gene is set forth in SEQ ID NO: 5;
preferably, the biomaterial is an expression cassette, a transposon, a plasmid vector, a phage vector, or a virus vector.

24. A recombinant DNA or biomaterial for use in preparing a keto acid, wherein the recombinant DNA or biomaterial
comprises L-aldolase and first dehydratase genes; or, the recombinant DNA or biomaterial comprises a D-aldolase gene, a racemase gene, and a second dehydratase gene;
preferably, the L-aldolase gene is selected from one or more of ltaE, ltaE_Pp, psald, dhaa, CC_3093, fbaA, itaA, glyA, or URA1, and is preferably ltaE_Pp or ltaE, wherein more preferably, the nucleotide sequence of the ltaE_Pp or ltaE gene is set forth in SEQ ID NO. 1 or SEQ ID NO. 2; the first dehydratase gene is selected from one or more of ilvA, tdcB, TDH, CHA1, TD2, A8H32_14290, Saut _1089, and C0627_08730, and is preferably ilvA or A8H32_14290, wherein more preferably, the nucleotide sequence of the ilvA or A8H32_14290 gene is set forth in SEQ ID NO. 3 or SEQ ID NO. 4;
alternatively, the D-aldolase gene is selected from one or more of A0A1C9ZZ39_CHLRE, tasS, dna, cghG, folB, guaB, dus, dhaa, bhcC, NCTC12151_01614, A4G23_03658, OJAG_33340, and GGC03_18995, and is preferably A0A1C9ZZ39_CHLRE, wherein more preferably, the nucleotide sequence of the A0A1C9ZZ39_CHLRE gene is set forth in SEQ ID NO. 7; the racemase gene is selected from one or more of ILE2E_LENBU, agiA, puuE, PS659_05479, HRbin10_02390, CVS47_02795, HRbin08_01795, and MJ8_44540, and is preferably ILE2E_LENBU, wherein more preferably, the nucleotide sequence of the ILE2E_LENBU gene is set forth in SEQ ID NO. 8; the second dehydratase gene is selected from one or more of ilvA, tdcB, TDH, CHA1, TD2, A8H32_14290, Saut _1089, and C0627_08730, wherein more preferably, the nucleotide sequence of the ilvA gene is set forth in SEQ ID NO. 3;
preferably, the biomaterial is an expression cassette, a transposon, a plasmid vector, a phage vector, or a virus vector.

25. The recombinant DNA or biomaterial as claimed in claim 23 or 24, wherein the keto acid comprises, but is not limited to, a keto acid having the following general formula: 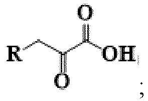 wherein the structural formula of R may be 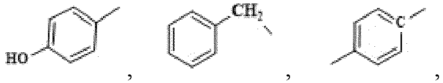 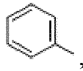 (CH₃)₂CH₂-, (CH₃)C-, CH₃-, 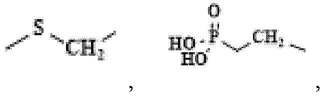 etc.

26. Use of the recombinant microorganism for preparing a keto acid as claimed in any one of claims 19-22, and the recombinant DNA or biomaterial for preparing a keto acid as claimed in any one of claims 23-25 for manufacturing a keto acid.

27. Use of the recombinant microorganism for preparing a keto acid as claimed in any one of claims 19-22, and the recombinant DNA or biomaterial for preparing a keto acid as claimed in any one of claims 23-25 for manufacturing an amino acid or an amino acid derivative.

28. A method for preparing a glycol organic substance, wherein the glycol organic substance is generated using the β-hydroxy-α-amino acid obtained by the method as claimed in any one of claims 1-19 in the presence of a deaminase, a decarboxylase, and a reductase.
